# EUROPEAN PATENT APPLICATION

(11) **EP 4 478 840 A1**
(43) Date of publication of application: **18.12.2024**
(21) Application number: 23179545.1
(22) Date of filing: 15.06.2023
(51) Int. Cl.: H05H 1/24, A61L 2/14, H01J 37/32

(54) **A DIELECTRIC BARRIER DISCHARGE PLASMA REACTOR COMPRISING AN IMPELLER**

(71) Applicant: Universiteit Antwerpen, 2000 Antwerpen (BE)
(72) Inventor: Bogaerts, Annemie, 2160 Wilrijk (BE); Lin, Abraham, 2160 Wilrijk (BE); Nguyen Thuan, Dao, 2160 Wilrijk (BE); Privat Maldonado, Angela, 2160 Wilrijk (BE)
(74) Representative: De Clercq & Partners

(57) **Abstract**

There is described a dielectric barrier discharge plasma reactor (1) comprising an impeller (60) configured for moving one or more items (20) during at least part of the plasma treatment of the one or more items (20).

## Description

### Technical field

The technical field is related to the field of methods and devices for cleaning, sterilizing, disinfecting, inactivating pathogens, decontaminating, ... of one or more items. More particularly the technical field is related to such methods and devices making use of a plasma treatment. The items could for example be medical items, items within or without sterile packages, clothing items, bedding items, food items, agricultural items, pharmaceutical items, aero-space items,... .

### Background

There is for example known from WO2019215741A1 a device for disinfecting herbs using plasma. The device is configured, upon receiving from a power source a suitable radio frequency electromagnetic power, to apply a plasma generating electromagnetic field for producing cold plasma in a plasma generation region of a sealable vessel. The plasma generated in this plasma generation region of the vessel is then allowed into a treatment region of the vessel comprising the product. The plasma generation region has fluid connectivity with this treatment region comprising the product to allow the plasma to flow towards the treatment region for treatment of the product. The amount or quantity of product, or in other words items, in the treatment region is limited. Further the application of the plasma treatment to the different items or product in different areas in the treatment region cannot be guaranteed and is dependent on the flow of the plasma through the items or product in the treatment region. Further, an increase in the amount of product, or in other words the number of items will increase hindrance to the flow of the plasma through the treatment region, thereby reducing the efficiency of the plasma treatment. WO2019215741A1 suggests making use of a gas flow or a reduced pressure in the vessel, for example generated by means of a gas pump to improve plasma flow from the plasma generation region to the treatment region. However, this increases complexity of the device and creates the need for a sterile gas flow and/or creates a risk of further contamination by means of the gas being used. Further, the gas that has flown through the treatment region could be contaminated and/or comprise hazardous substances, such as for example chemical or biological agents, and could thus create further risks and/or complexity by further treatment necessities when released from the system.

A further device is for example known from US8771595B2 which describes a sterilization method that includes placing a substance to be sterilized in a rotating chamber, for example a cylindrical drum, and exposing the substance to a radio frequency plasma. In order to further promote the mixing of the substance and plasma there is a generated magnetic field that produces a force on the substance in a direction opposite to the rotational direction of the chamber. Further also the chamber may have a gas permeable wall or the substance may be exposed to acoustic shock waves produced by a modulating radio frequency generator to improve the distribution of plasma amongst the substance. However, it is clear that all these means increase complexity of the method and device and exhibit the above-mentioned problems when a gas flow is used. The effect of the electromagnetic field, gas, acoustic shock waves will also vary considerably depending on the type of substance, product or items being treated.

Further plasma treatment methods and devices, for example for sterilizing or disinfecting medical or hygiene articles are for example known from WO2018/05715 or EP2756739 which require a gas to circulate the plasma from a plasma chamber to a treatment area comprising the substance, product or objects to be treated. However, it is clear that all these means increase complexity of the method and device and exhibit the above-mentioned problems when a gas flow is used.

There is thus a need for an improved device and method that overcomes the above-mentioned problems and is able to provide a more efficient, simpler, reliable, flexible and more uniform plasma treatment of one or more items.

### Summary

Therefore, according to a first aspect, there is provided a dielectric barrier discharge plasma reactor comprising:
- a reactor container configured for comprising one or more items during a plasma treatment; and
- at least one first electrode, at least one second electrode and at least one insulating dielectric barrier separating the at least one first electrode and the at least one second electrode, configured to generate plasma in the reactor container comprising one or more items upon an electrical discharge between the at least one first electrode and the at least one second electrode during a plasma treatment of the one or more items,
CHARACTERISED IN THAT
the dielectric barrier discharge plasma reactor further comprises an impeller protruding at least partly into the reaction container and configured for moving the one or more items during at least part of the plasma treatment of the one or more items.

In this way the plasma treatment can be applied efficiently without the need to circulate it from a generation area to the area where the items are treated. Further a more uniform distribution of the plasma amongst and along the items is assured by means of the action of the impeller moving the items in the reaction container. Such an approach is very flexible and robust and can be applied to a large variety of items and/or reaction containers.

According to an embodiment, there is provided a dielectric barrier discharge plasma reactor, wherein the impeller comprises or consists of at least one element configured for:
- movement during at least a part of the plasma treatment of the one or more items;
- movement during at least a part of the plasma treatment with respect to the reactor container; and/or
- imparting movement to at least a part of the one or more items by means of a mechanical force.

In this way a simple and efficient mixing of the items and the plasma is realized in the area where the plasma is generated, thereby allowing a more uniform generation and application of the plasma.

According to an embodiment, there is provided a dielectric barrier discharge plasma reactor, wherein the element comprises or consists of one or more of the following:
- the at least one first electrode;
- the at least one second electrode;
- the at least one insulating dielectric barrier.

In this way the plasma can be generated in a simple, more efficient and more uniform way and applied in a more efficient and uniform way to the one or more items in the reaction container during the plasma treatment.

According to a further embodiment, there is provided in a dielectric barrier discharge plasma reactor, further comprising an actuator coupled to the impeller and operable to move the at least one element during at least a part of the plasma treatment of the one or more items.

In this way the mechanical movement of the impeller can be realized in a simple and efficient way.

According to an embodiment, there is provided in a dielectric barrier discharge plasma reactor, comprising one or more insulating dielectric elements, which are different from the one or more items, provided in the reactor container together with the one or more items.

According to an embodiment there is provided in a dielectric barrier discharge plasma reactor comprising a mixture of insulating dielectric elements, such as for example beads, balls, blocks, particles, ... , with the one or more of the items. Preferably the dielectric elements and the one or more items are arranged in a closed-pack arrangement in the reaction container.

In this way plasma generation in the reactor container is supported by the presence of the dielectric elements and generated in a more efficient way.

According to an embodiment, there is provided a dielectric barrier discharge plasma reactor, wherein the dielectric barrier discharge plasma reactor comprises or consists of a cylindrical dielectric barrier discharge plasma reactor comprising:
- cylindrical reactor container;
- at least one first electrode extending at least partly inside the cylindrical reactor container;
- at least one second electrode extending at least partly around the cylindrical reactor container; and/or
- at least one impeller comprising or consisting of an element configured for moving one or more items, provided in the cylindrical reactor container between the at least one first electrode and the at least one second electrode, during at least part of the plasma treatment of the one or more items.

In this way, a simple and efficient operation of the impeller is made possible resulting in a good mixing of the items and/or the generated plasma and thus a more efficient and uniform application of the plasma treatment.

According to an embodiment, there is provided a dielectric barrier discharge plasma reactor, wherein:
- the at least one second electrode is arranged stationary; and/or
- the at least one impeller is configured for movement with and/or around the at least one first electrode during at least part of the plasma treatment of the one or more items.

In this way a simple, efficient and reliable construction is enabled.

According to an embodiment, there is provided a dielectric barrier discharge plasma reactor, wherein:
- the movement of the element of the impeller is a rotational movement with and/or around the at least one first electrode;
- the impeller comprises and/or consists of an element comprising a helical shape.

In this way a simple, efficient movement of an impeller is realized which provides for optimized mixing of the items and/or the plasma.

According to an embodiment, there is provided a dielectric barrier discharge plasma reactor, wherein the element of the at least one impeller comprises and/or consists of:
- the at least one first electrode; and/or
- the at least one insulating dielectric barrier.

In this way the impeller design is integrated with and aids with the generation of the plasma in the area and/or at the location where the items are being present and mixed in the reactor container.

According to an embodiment, there is provided a dielectric barrier discharge plasma reactor assembly comprising the dielectric barrier discharge plasma reactor according to the first aspect, wherein the assembly further comprises:
- one or more items provided in the reactor container.

According to a further embodiment, there is provided a dielectric barrier discharge plasma reactor assembly, comprising one or more insulating dielectric elements, which are different from the one or more items, provided in the reactor container together with the one or more items.

According to an embodiment there is provided a dielectric barrier discharge plasma reactor assembly comprising a mixture of insulating dielectric elements, such as for example beads, balls, blocks, particles, ... , with the one or more of the items. Preferably the dielectric elements and the one or more items are arranged in a closed-pack arrangement in the reaction container.

In this way plasma generation in the reactor container is supported by the presence of the dielectric elements and generated in a more efficient way.

According to a second aspect, there is provided a method of operating a dielectric barrier discharge plasma reactor according to the first aspect, comprising the steps of:
- providing one or more items in the reactor container; and
- moving the one or more items by means of the impeller during at least part of the plasma treatment of the one or more items.

According to an embodiment there is provided a method further comprising the step of:
- moving the at least one element of the impeller during at least a part of the plasma treatment of the one or more items; and/or
- the at least one element of the impeller imparting movement to at least a part of the one or more items by means of a mechanical force.

According to an embodiment there is provided a method further comprising the step of:
- operating an actuator coupled to the impeller to move the element during at least a part of the plasma treatment of the one or more items.

According to an embodiment, there is provided a method further comprising the step of:
- providing one or more insulating dielectric elements, which are different from the one or more items, provided in the reactor container together with the one or more items.

According to an embodiment there is provided in a method further comprising the step of:
- providing a mixture of insulating dielectric elements, such as for example beads, balls, blocks, particles, ... , with the one or more of the items. Preferably arranging the dielectric elements and the one or more items in a closed-pack arrangement in the reaction container.

In this way plasma generation in the reactor container is supported by the presence of the dielectric elements and generated in a more efficient way.

According to an embodiment there is provided a method further comprising the steps of:
- providing the one or more items in the cylindrical reactor container between the at least one first electrode and the at least one second electrode; and/or
- moving the one or more items during at least part of the plasma treatment of the one or more items by means of the at least one element of the at least one impeller.

According to an embodiment there is provided a method further comprising the steps of:
- keeping the at least one second electrode stationary; and/or
- moving the at least one impeller with and/or around the at least one first electrode during at least part of the plasma treatment of the one or more items.

According to an embodiment there is provided a method further comprising the step of:
- moving the at least one element of the impeller by means of a rotational movement with and/or around the at least one first electrode.

According to an embodiment there is provided a method further comprising the steps of:
- providing the element of the at least one impeller comprising and/or consisting of:
- the at least one first electrode; and/or
- the at least one insulating dielectric barrier.

According to a further embodiment there is provided a method according to the first aspect for cleaning and/or sterilizing and/or disinfecting and/or inactivating pathogens and/or decontaminating the one or more items in the reactor container during the plasma treatment.

It is clear that further alternative embodiments and/or combinations are possible, more particularly also with reference to embodiments of other aspects, without departing from the scope of the appended claims.

### Brief description of the drawings

Merely by way of example exemplary embodiments will be described by means of the Figures in which:
- Figure 1 shows an embodiment of a dielectric barrier discharge plasma reactor;
- Figure 2 schematically shows the fragment II in Figure 1 in more detail;
- Figure 3 shows an alternative embodiment similar to that of Figure 1;
- Figure 4 shows a schematic view of the cross section IV-IV in Figure 1;
- Figure 5 shows an alternative embodiment similar to that of Figure 4;
- Figure 6 shows a perspective view of an embodiment of an impeller similar to that of the embodiment of Figure 1;
- Figure 7 shows a perspective view of an embodiment of an impeller similar to that of the embodiment of Figure 3; and
- Figure 8 shows a perspective view of an alternative embodiment of an impeller, similar to the embodiments of Figure 6 and 7.

### Detailed description

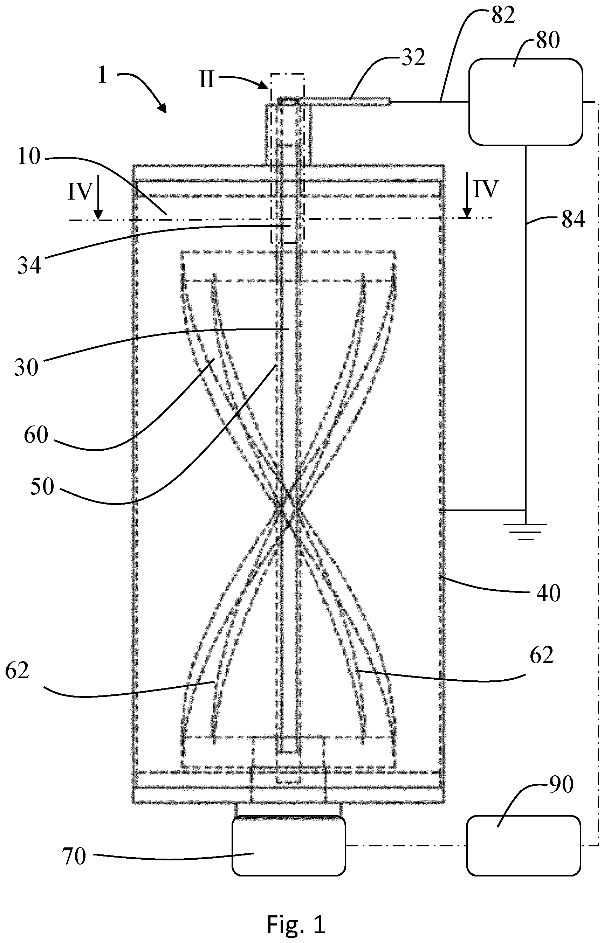

Figure 1 shows an exemplary embodiment of a dielectric barrier discharge plasma reactor 1. As shown the dielectric barrier discharge plasma reactor 1, which will also be referred to as the DBD plasma reactor 1 or as the reactor 1, comprises a reactor container 10 and a first electrode 30 and a second electrode 40. Although, according to the embodiment of Figure 1 there is only shown one first electrode 30 and one second electrode 40, it is clear that alternative embodiments are possible with any suitable number of first electrodes and/or second electrodes, such as for example a plurality, two, three, four or more first electrodes and/or second electrodes. In general, the DBD plasma reactor 1 comprises at least one first electrode 30 and at least one second electrode 40.

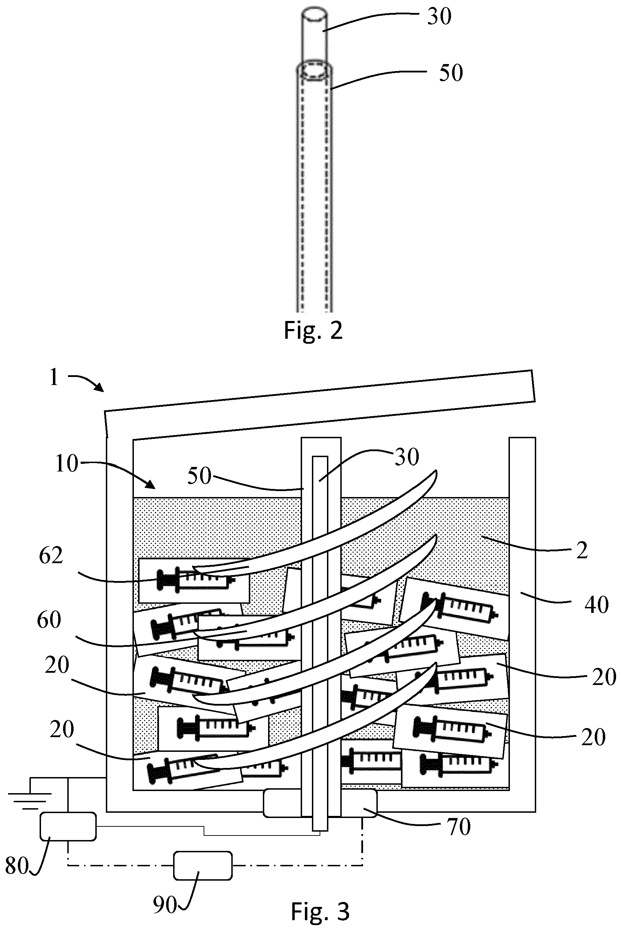

As also shown further below with reference to for example Figure 3, the DBD plasma reactor 1 comprises a reactor container 10 configured for comprising one or more items 20 during a plasma treatment. As for example shown in Figure 3, these one or more items 20 in the reactor container 10 are in a region of the reactor container 10 where the plasma 2 is generated and is present during a plasma treatment, or in other words when the DBD plasma reactor 1 is activated. It is clear that similarly as in the embodiment of Figure 3, also one or more items 20 could be placed into the reactor container 10 of the embodiment of Figure 1. As described further below, during a plasma treatment, or in other words upon activation of the DBD plasma reactor 1, similarly, also plasma 2 would be generated and be present in the area of the one or more items 20 in the reactor container 10.

According to an alternative embodiment, not shown, optionally, there can be provided also other elements than the one or more items in the reactor container. According to such an embodiment, there are for example provided one or more insulating dielectric elements, which are different from the one or more items 20, in the reactor container together with the one or more items 20. In this way there could for example be provided a mixture of insulating dielectric elements, such as for example beads, balls, blocks, particles, ... , with the one or more of the items 20 in the reactor container 10. Preferably, the dielectric elements and the one or more items are arranged in a closed-pack arrangement in the reaction container 10. In this way plasma generation in the reactor container is supported by the presence of the dielectric elements and generated in a more efficient way. However it is clear that numerous alternative embodiments are possible. The insulating dielectric elements comprise and/or consist of and/or substantially comprise and/or substantially consist of an insulating dielectric material.

According to the embodiments shown in Figure 1 and 3, it is clear that the first electrode 30 and the second electrode 40 are separated by an insulating dielectric barrier 50. They are arranged in such a way to generate plasma 2 in the reaction container 10 comprising one or more items 20 upon an electrical discharge between the first electrode 30 and the at least one second electrode 40.

Figure 2 shows an embodiment of the dielectric barrier 50 surrounding the first electrode 30, at least the part of the first electrode 30 present in the reaction container 10, indicated as fragment II of Figure 1 in further detail. It is however clear that further alternative embodiments are possible for separating the first electrode 30 and the second electrode 40 by an insulating dielectric barrier 50.

In general, the DBD plasma reactor 1 thus comprises at least one first electrode 30, at least one second electrode 40 and at least one insulating dielectric barrier 50 separating the at least one first electrode 30 and the at least one second electrode 40, configured to generate plasma in the reactor container 10 comprising one or more items 20 upon an electrical discharge between the at least one first electrode 30 and the at least one second electrode 40 during a plasma treatment of the one or more items 20.

As further shown in Figure 1, the dielectric barrier discharge plasma reactor 1 further comprises an impeller 60 protruding at least partly into the reaction container 10 and configured for moving the one or more items 20 during at least part of the plasma treatment of the one or more items 20. The embodiment of the impeller 60 will be described in further detail below, however it is clear that numerous alternative embodiments of such an impeller 60 are possible and that such an impeller 60 can for example also be referred to as a mixer.

The alternative embodiment of the DBD plasma reactor shown in Figure 3 comprises a similar embodiment as described with reference to the embodiment of Figure 1 and similar elements are provided with similar references and are similar in structure as in function as described with reference to Figure 1 and vice versa.

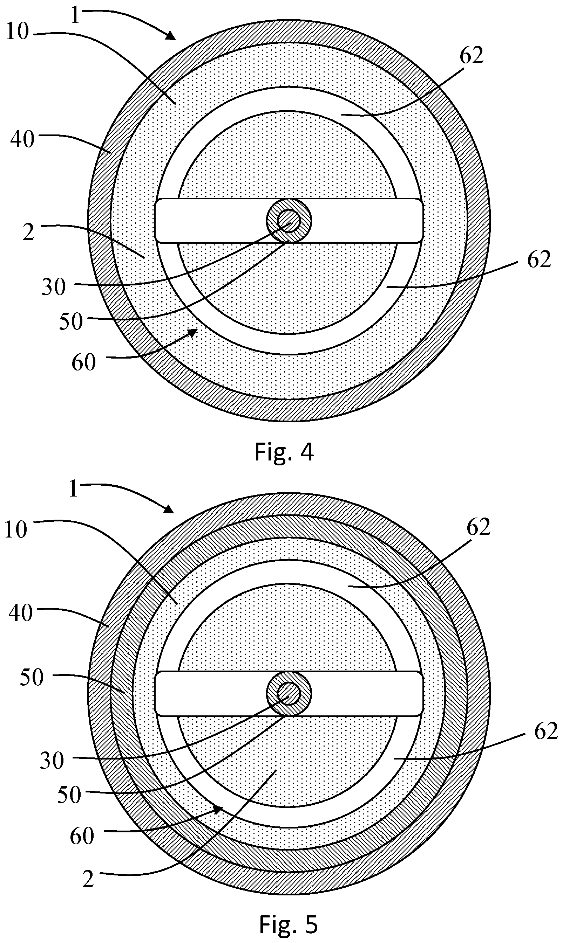

According to the embodiment shown in Figure 1, the dielectric barrier discharge plasma reactor 1 comprises a cylindrical reactor container 10. This is further illustrated by the schematic representation of the cross section IV-IV in Figure 1 shown in Figure 4. As further shown, according to this embodiment, the DBD plasma reactor 1 comprises a second electrode 40 that extends around the cylindrical reactor container 10. In other words, according to such an embodiment the second electrode 40 forms, is part of and/or is attached to a cylindrical shell of the plasma reactor 1. As further shown, according to this embodiment, the first electrode 30 extends at least partly inside the cylindrical reactor container 10. As shown, according to this embodiment, the first electrode 30 is a longitudinal or elongate electrode extending parallel to and/or along the central axis of the cylindrical reactor container 10. As further shown, according to the embodiment of Figure 1 and Figure 4, there is provided an insulating dielectric barrier 50 at least around the part of the first electrode 30 protruding into the reactor container 10. As shown, it forms a cylindrical mantle for the first electrode 30 which has a round cross section. However, it is clear that alternative embodiments are possible in which at least one first electrode 30 extends at least partly inside the cylindrical reactor container 10 and at least one second electrode 40 extends at least partly around the cylindrical reactor container 10.

The embodiment of Figure 4 illustrates a type of DBD reactor 1 known as a cylindrical one-sided DBD reactor.

A cylindrical DBD reactor comprises for example a cylindrical quartz tube that acts as a dielectric barrier, surrounding an inner electrode and surrounded by an outer electrode. Preferably the first or positive electrode is placed inside the quartz reactor, and it can for example be made of stainless steel, tungsten, among others. The negative or second electrode is preferably placed on the outside of the quartz reactor and can for example be made of silver paste, stainless steel mesh, aluminium foil, copper thin film, among others.

A one-sized DBD reactor comprises at least one first electrode 30 and second electrode 40, where only one of the first or second electrode is covered by an insulating dielectric barrier 50. According to the embodiment of Figure 4, this is the first electrode 30. The other electrode, according to the embodiment of Figure 4, the second electrode 40 is exposed to the plasma. This asymmetric configuration can lead to the uncovered electrode being directly exposed to the plasma and/or the items 20 in the reaction container 10. One-sided DBD reactors can have the benefit of an enhanced plasma-surface interaction.

According to alternative embodiments, there can for example be provided a two-sided DBD plasma reactor 1, such as shown in Figure 5, in which similar elements are shown with similar references and are similar with respect to their structure and function as for the embodiment of Figure 4. However different from the embodiment of Figure 4, according to the embodiment of figure 4, the DBD plasma reactor 1 comprises at least one first electrode 30 and second electrode 40, which are both covered by an insulating dielectric barrier 50. According to such an embodiment, the plasma and the items 20 in the reaction container 10 are isolated from the first electrode 30 and second electrode 40, protecting the electrodes and/or the items from any undesired interaction.

It is clear that instead of a cylindrical DBD reactor 1 with a circular cross section, it is possible to make use of any other suitable shape for the DBD reactor 1, for example with any suitable curved and/or polygonal cross section.

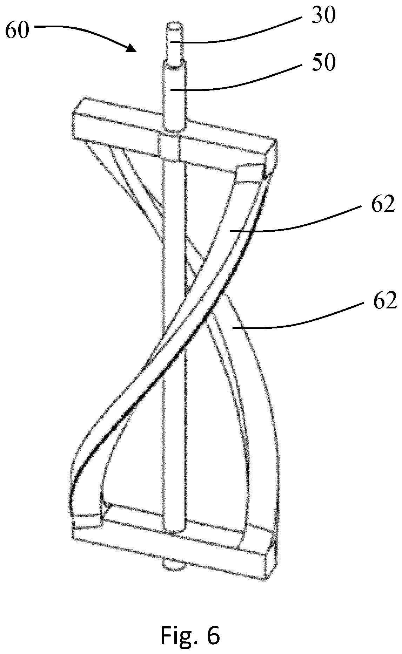

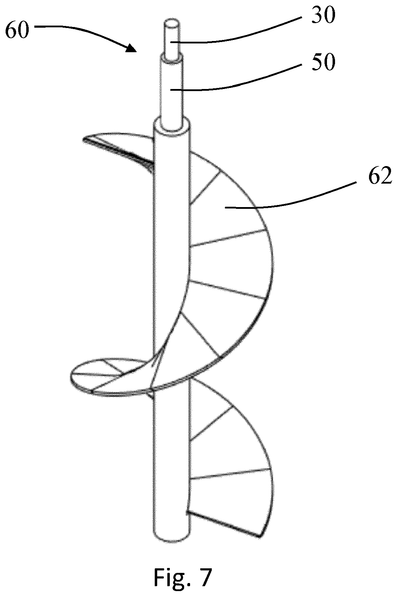

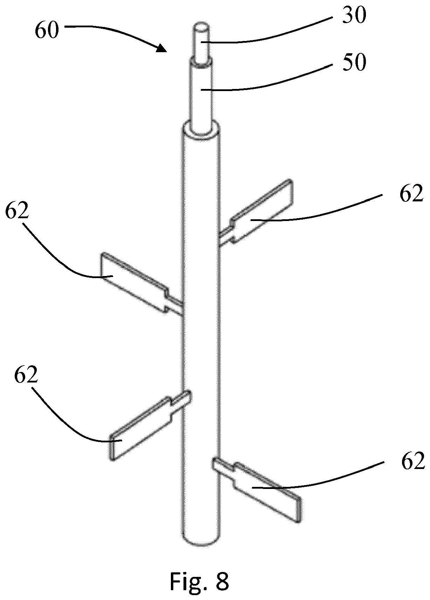

As further shown in with the perspective view of Figure 6, the impeller 60 of the embodiment of Figure 1 and Figure 6 for example comprises two elements 62 comprising a helical shape. It is clear that alternative embodiments are possible, such as for example similar to the embodiment of Figure 3 in which the impeller 60 comprises one element 62 comprising a helical shape, such as for example shown in the perspective view of the embodiment of Figure 7, showing an impeller 60 similar as in the embodiment of Figure 3. It is however clear that with respect to the shape and/or number of elements 62 of the impeller 60 numerous alternative embodiments are possible. According to such embodiments, the DBD reactor 1 comprises at least one impeller 60 comprising or consisting of an element 62 configured for moving one or more items 20. These one or more elements 62 of the impeller are provided in the cylindrical reactor container 10 between the at least one first electrode 30 and the at least one second electrode 40. It is clear that these elements 62 are configured to impart the movement on the one or more items 20 during at least part of the plasma treatment of the one or more items 20. According to further embodiments, the one or more elements 62 could for example comprise or consist of one or more vanes, longitudinal bars, plates, fingers, ... configured for imparting movement to the one or more items 20 when the impeller 60 is moved, preferably rotated within the reactor container 10. Such an embodiment has for example been illustrated by means of the embodiment of Figure 8. Similar features have been provided with similar references and function in a similar way as described herein. In other words, according to such embodiments, the impeller 60 comprises one or more elements 62 configured for mixing the one or more items 20 within the reaction container 10 upon movement, preferably rotation, of the impeller 60.

According to the embodiments shown in Figure 1 and Figure 3, the dielectric barrier discharge plasma reactor comprises a second electrode 40 which is arranged stationary. The impeller 60 is configured for movement with and/or around the at least one first electrode 30 during at least part of the plasma treatment of the one or more items 20. According to some embodiments, the impeller 60 is configured for rotational movement with and/or around the first electrode 30. According to the embodiment shown, a suitable rotary actuator 70, such as for example an electromotor or any other suitable actuator is coupled to the impeller 60 for imparting the rotational movement of the impeller 60 within the reactor container 10. According to a preferred embodiment the at least one element 62 of the at least one impeller 60 comprises and/or consists of the at least one insulating dielectric barrier 50. This could simply be realised by manufacturing the impeller 60 from a suitable insulating dielectric barrier 50 and inserting the first electrode 30 into it. It is however clear that numerous alternative embodiments are possible in which the impeller, and/or the elements 62 of the impeller further comprise at least one first electrode 30, in which the impeller 60 is just a separate element from the first electrode 30 and/or the dielectric insulating barrier 50, ....

According to the embodiments shown in Figure 1 and 3, the top and/or bottom plane of the cylindrical reactor container 10 might comprise and/or consist of a suitable electrical isolator and/or at least a part of the second electrode 40, through which the first electrode 30 and/or the insulating dielectric barrier 50 is inserted into the reactor container 10, and through which the actuator 70 is operatively coupled to the impeller 60 for imparting the movement of the impeller during operation, or in other words, during activation of the DBD plasma reactor 1.

It is clear that still further embodiments are possible in which the dielectric barrier discharge plasma reactor 1 an actuator 70 coupled to the impeller 60 and operable to move the at least one element 62 during at least a part of the plasma treatment of the one or more items 20.

According to still further embodiments the dielectric barrier discharge plasma reactor could comprise at least one element 62 comprising or consisting of at least one first electrode 30 or second electrode 40 and/or at least one insulating dielectric barrier 50.

As for example clearly shown in Figure 3, there is provided an embodiment of a dielectric barrier discharge plasma reactor assembly comprising the dielectric barrier discharge plasma reactor 1 and further also comprising the one or more items 20 provided in the reactor container 10. The one or more items 20 could be inserted and/or extracted from the reactor container 10 in any suitable way.

As further shown, optionally the dielectric barrier discharge plasma reactor assembly could further comprise a suitable high voltage power supply 80 coupled to the at least one first electrode 30 via a first conductor 82 and the at least one second electrode 40 via a second conductor 84. Further, there could optionally be provided a suitable controller 90 configured to control and/or activate the high voltage power supply 80 and the actuator 70 during at least a part of the plasma treatment.

It is clear that in this way there is provided a Method of operating a dielectric barrier discharge plasma reactor according to for example the embodiments described above, comprising the steps of providing one or more items 20 in the reactor container 10. This could for example be realised by opening up the top cover of the cylindrical reactor container 10 such as for example illustrated schematically in Figure 3. However, it is clear that any other suitable way of providing the items 20 in the reactor container 10 is possible. After introduction of the items 20 in the reactor container 10, the method can be continued by means of the step of moving the one or more items 20 by means of the impeller 60 during at least part of the plasma treatment of the one or more items 20. It is thus clear that the impeller 60, according to the embodiments shown moves the at least one element 62 of the impeller 60 during at least a part of the plasma treatment of the one or more items 20. In this way, according to the embodiments shown the at least one element 62 of the impeller 60 imparts movement to at least a part of the one or more items 20 by means of a mechanical force. In this way there is thus provided a method for cleaning and/or sterilizing and/or disinfecting and/or decontaminating the one or more items 20 in the reactor container 10 during the plasma treatment.

For the embodiments described above, it is clear that this step of movement of the impeller 60, which is provided with at least one element 62 comprising and/or consisting of the at least one first electrode 30 and/or the at least one insulating dielectric barrier 50, preferably comprises moving the at least one element 62 of the impeller 60 by means of a rotational movement with and/or around the at least one first electrode 30. According to such embodiments, preferably the actuator 70 is operated to move the element 62 during at least a part of the plasma treatment of the one or more items 20, thereby realizing a mixing of the items 20 and the plasma inside the reaction container 10 mechanically. As further shown in for example Figure 1, preferably, when at least a part 34 of the first electrode 30 is rotated with the impeller 60, there is provided conductive coupling part 32 configured to electrically couple the rotating first electrode 30 to a stationary conductor 82 coupled to the high voltage source 80. Any suitable element that in general functions similar as a slip ring providing electric contact between a rotating or movable conductor and a stationary conductor may be used.

Although in the embodiments described above, the at least one second electrode 40 is kept stationary; and the at least one impeller 60 is moved with and/or around the at least one first electrode 30 during at least part of the plasma treatment of the one or more items 20. It is clear that alternative embodiments are possible in which the at least one first electrode 30 is kept stationary and in which the at least one impeller 60 is moved with and/or inside the at least one second electrode 40. According to still further alternative embodiments, there could be provided multiple impellers 60 in a reaction container 10, of any combination of the types described above.

It is clear that still further embodiments are possible in which there are provided one or more items 20 in the cylindrical reactor container 10 between the at least one first electrode 30 and the at least one second electrode 40 and then the one or more items 20 are moved during at least part of the plasma treatment of the one or more items 20 by means of the at least one element 62 of the at least one impeller 60.

Still further embodiments are possible of the method of operation and/or of the of the impeller 60 and/or further embodiments of the dielectric barrier discharge plasma reactor 1 in which the impeller 60 comprises or consists of at least one element 62 configured for:
- movement during at least a part of the plasma treatment of the one or more items 20;
- movement during at least a part of the plasma treatment with respect to the reactor container 10; and/or
- imparting movement to at least a part of the one or more items 20 by means of a mechanical force.

According to exemplary embodiments for example the impeller 60 could be operated for movement during the entire plasma treatment of the one or more items 20. For example, the impeller 60 could start rotation before or when the plasma generation is started and stop after or when the plasma treatment of the one or more items 20 is ended. According to other embodiments, the movement of the impeller 60 could be initiated only after the plasma generation was started, and/or stopped before the plasma generation was ended. Still further embodiments are possible in which the plasma treatment comprises for example a one or more plasma generation phases during which plasma is generated and/or a one or more mixing phases during which the impeller 60 is moved. It is clear that, according to such embodiments, these one or more plasma generation phases and/or mixing phases could at least partly overlap and/or proceed intermittently and/or in any other suitable combination. It is further clear that the number of such mixing and plasma generation phases of the plasma treatment do not necessarily need to be identical. For example, according to one embodiment, there could be a single plasma generation phase spanning the entire plasma treatment combined with a plurality of intermittent mixing phases. According to another embodiment, there could be a single mixing phase spanning the entire plasma treatment combined with a plurality of intermittent plasma generation phases. According to still further embodiments the plasma treatment could comprise at least one mixing phase, which does not overlap with a plasma generation phase, for example mixing the items 20 before and/or after a plasma generation phase. According to other embodiments such a mixing phase could at least partly overlap one or more plasma generation phases, such as for example continuing the mixing of items 20 by the impeller 60 after a plasma generation phase has started or beginning the mixing of items 20 before a plasma generation phase has ended. It is clear that still further alternative embodiments and/or combinations thereof are possible, in which the impeller 60 comprises or consists of at least one element 62 configured for imparting movement to at least a part of the one or more items 20 by means of mechanical force during one or more of such mixing phases of a plasma treatment, wherein in the plasma treatment comprises at least one plasma generation phase during which plasma is generated in the reaction container 10 in which the impeller 60 is arranged. According to certain embodiments a plasma treatment comprising any suitable sequence of at least partly overlapping and/or non-overlapping mixing phases and/or plasma generation phases are possible.

In the context of this description, the one or more of the following clarifying definitions might be used.

One or more items could be used synonymously with one or more substances and/or one or more products and/or any suitable combinations thereof.

Dielectric-barrier discharge or DBD is generally known to the skilled person as the electrical discharge between two electrodes separated by an insulating dielectric barrier. The DBD plasma generation process typically makes use of a suitable high voltage alternating current and/or pulsed current, such as for example pulsed direct current or pulsed DC, such as for example positive or negative pulsed DC, of which typically the frequency ranges from 50Hz to 100kHz. However, alternative DBD processes are known, which extend the frequency range all the way down to direct current or DC. A suitable high voltage source for a DBD discharge typically is in the range of 1kV to 30kV.

An electrode is an electrical conductor configured to provide the high voltage current to the reactor container. Typically, an electrode comprises and/or consists of a metal conductor.

An insulating dielectric barrier, also known as a dielectric or a dielectric medium comprises and/or consists and/or substantially comprises and/or substantially consists of a dielectric material, which is an electrical insulator that can be polarised by an applied electric field. When such an insulating dielectric barrier experiences an electric field, electric charges do not flow through the insulating dielectric barrier as they do in an electrical conductor, because such an insulating dielectric barrier does not comprise sufficiently loosely bound, or free, electrons that may drift through the material. Instead, in such an insulating dielectric barrier there is caused a shift, typically a slight shift, from the average equilibrium positions of the electrons, thereby causing dielectric polarisation. Typically, an insulating dielectric barrier comprises and/or consists of one or more of the following solid dielectric materials: glass, quartz, plastic, ceramics, polymers, an acrylic resin, .... An insulating dielectric element comprises and/or consists and/or substantially comprises and/or substantially consists of a dielectric material.

Plasma is generally known to the skilled person as a separate state of matter, in addition to the other states of matter: solid, liquid, and gas. Plasma can for example be defined as a state of matter in which an ionized substance becomes highly electrically conductive to the point that long-range electric and magnetic fields dominate its behaviour. Although plasma is similar to the gas phase in that both assume no definite shape or volume and is sometimes referred to as merely an ionized gas, it is clear to a skilled person that plasma is distinct from the other states of matter. For example, the electric conductivity is higher, typically several orders of magnitude higher, than that of gases which are excellent insulators up to electric field strengths of tens of kilovolts per centimetre.

Plasma generated by means of dielectric barrier discharge or DBD, such as for example by means of a DBD plasma reactor, can also be referred to as DBD plasma.

DBD is generally known as a method for generating DBD plasma at a relatively low temperature, for example at or around room temperature, or in other words a method for generating non-thermal plasma or NTP. This means that, although in a DBD, the average temperature of high-energy electrons can be very high, for example over the range of 10000-100000 K, the actual gas temperature is at or close to the environment temperature, such as for example environment or room temperature +/- 5°C, or a temperature in the range of 25°C - 125°C.

DBD is also generally known as a method for generating DBD plasma without the need for a low-pressure gas for example around 1/1000th of atmospheric pressure. DBD is generally known as method for generating DBD plasma at or around atmospheric pressure, or in other words as an atmospheric pressure plasma. At or around atmospheric pressure could for example be defined as the atmospheric pressure in the environment surrounding a DBD plasma reactor +/- 20%

Plasma cleaning and/or sterilization and/or disinfection for example refers to the removal, inactivation, reduction of infectivity, disinfection, ... of impurities, contaminants, bacteria, viruses, fungi, ... from items, surfaces, objects, ... for example through the use of DBD plasma.

DBD plasma can for example be generated from gaseous species and/or mixes thereof, such as for example air, hydrogen, argon, oxygen, nitrogen, helium, carbon dioxide, methane, ammonia, ... and their mixtures.

A mechanical force involves physical contact with another object, item, substance, product, structural element, ....

It is to be understood that this invention is not limited to a particular device or method described, since such device or method may, of course, vary. It is also to be understood that the terminology used herein is not intended to be limiting, since the scope of the present invention will be limited only by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear per se, by means of further exemplification, the term encompasses inter alia a reference to any one of said members, or to any two or more of said members, such as, e.g., any ≥3, ≥4, ≥5, ≥6 or ≥7 etc. of said members, and up to all said members.

All references cited in the present specification are hereby incorporated by reference in their entirety. In particular, the teachings of all references herein specifically referred to are incorporated by reference.

Unless otherwise defined, all terms used in this specification, including technical and scientific terms, have the meaning as commonly understood by one of ordinary skill in the art to which this invention belongs. By means of further guidance, term definitions are included to better appreciate the teaching of the present invention.

In this specification, different aspects and embodiments are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description, reference is made to the accompanying drawings that form a part hereof, and in which are shown by way of illustration only of specific embodiments in which the invention may be practiced. Reference numerals affixed to respective elements merely exemplify the elements by way of example, with which it is not intended to limit the respective elements. Unless otherwise indicated, all figures and drawings in this document are not to scale and are chosen for the purpose of illustrating different embodiments of the invention. In particular the dimensions of the various components are depicted in illustrative terms only, and no relationship between the dimensions of the various components should be inferred from the drawings, unless so indicated.

It is to be understood that other embodiments may be utilised and structural or logical changes may be made without departing from the scope of the present invention. The detailed description, therefore, is not to be taken in a limiting sense, and the scope of the present invention is defined by the appended claims.

## Claims

1. A dielectric barrier discharge plasma reactor (1) comprising:
- a reactor container (10) configured for comprising one or more items (20) during a plasma treatment; and
- at least one first electrode (30), at least one second electrode (40) and at least one insulating dielectric barrier (50) separating the at least one first electrode (30) and the at least one second electrode (40), configured to generate plasma in the reactor container (10) comprising one or more items (20) upon an electrical discharge between the at least one first electrode (30) and the at least one second electrode (40) during a plasma treatment of the one or more items (20),
**CHARACTERISED IN THAT**
the dielectric barrier discharge plasma reactor (1) further comprises an impeller (60) protruding at least partly into the reaction container (10) and configured for moving the one or more items (20) during at least part of the plasma treatment of the one or more items (20).

2. A dielectric barrier discharge plasma reactor according to claim 1, wherein the impeller (60) comprises or consists of at least one element (62) configured for:
- movement during at least a part of the plasma treatment of the one or more items (20);
- movement during at least a part of the plasma treatment with respect to the reactor container (10); and/or
- imparting movement to at least a part of the one or more items (20) by means of a mechanical force.

3. A dielectric barrier discharge plasma reactor according to claim 2, wherein the element (62) comprises or consists of one or more of the following:
- the at least one first electrode (30);
- the at least one second electrode (40);
- the at least one insulating dielectric barrier (50).

4. A dielectric barrier discharge plasma reactor according to claim 2 or 3, further comprising:
- an actuator (70) coupled to the impeller (60) and operable to move the at least one element (62) during at least a part of the plasma treatment of the one or more items (20);
- and optionally one or more insulating dielectric elements, which are different from the one or more items (20), provided in the reactor container (10) together with the one or more items (20).

5. A dielectric barrier discharge plasma reactor according to any of the preceding claims, wherein the dielectric barrier discharge plasma reactor comprises or consists of a cylindrical dielectric barrier discharge plasma reactor comprising:
- cylindrical reactor container (10);
- at least one first electrode (30) extending at least partly inside the cylindrical reactor container (10);
- at least one second electrode (40) extending at least partly around the cylindrical reactor container (10); and/or
- at least one impeller (60) comprising or consisting of an element (62) configured for moving one or more items (20), provided in the cylindrical reactor container (10) between the at least one first electrode (30) and the at least one second electrode (40), during at least part of the plasma treatment of the one or more items (20).

6. A dielectric barrier discharge plasma reactor according to claim 6, wherein:
- the at least one second electrode (40) is arranged stationary; and/or
- the at least one impeller (60) is configured for movement with and/or around the at least one first electrode (30) during at least part of the plasma treatment of the one or more items (20),
and optionally, wherein:
- the movement of the element (62) of the impeller (60) is a rotational movement with and/or around the at least one first electrode (30);
- the impeller (60) comprises and/or consists of an element (62) comprising a helical shape; and/or
- the element (62) of the at least one impeller (60) comprises and/or consists of:
- the at least one first electrode (30); and/or
- the at least one insulating dielectric barrier (50).

7. A dielectric barrier discharge plasma reactor assembly comprising the dielectric barrier discharge plasma reactor according to any of the preceding claims, wherein the assembly further comprises:
- one or more items (20) provided in the reactor container (10);
- and optionally one or more insulating dielectric elements, which are different from the one or more items (20), provided in the reactor container (10) together with the one or more items (20).

8. A Method of operating a dielectric barrier discharge plasma reactor according to any of the preceding claims, comprising the steps of:
- providing one or more items (20) in the reactor container (10); and
- moving the one or more items (20) by means of the impeller (60) during at least part of the plasma treatment of the one or more items (20).

9. The method according to claim 8, wherein the method further comprises the step of:
- moving the at least one element (62) of the impeller (60) during at least a part of the plasma treatment of the one or more items (20); and/or
- the at least one element (62) of the impeller (60) imparting movement to at least a part of the one or more items (20) by means of a mechanical force.

10. The method according to claim 8 or 9, wherein the method further comprises the step of:
- operating an actuator (70) coupled to the impeller (60) to move the element (62) during at least a part of the plasma treatment of the one or more items (20);
- and optionally providing one or more insulating dielectric elements, which are different from the one or more items (20), in the reactor container (10) together with the one or more items (20).

11. The method according to one or more of the claims 8 to 10 for operating a dielectric barrier discharge plasma reactor according to one or more of the claims 5 to 7, wherein the method further comprises the steps of:
- providing the one or more items (20) in the cylindrical reactor container (10) between the at least one first electrode (30) and the at least one second electrode (40); and
- moving the one or more items (20) during at least part of the plasma treatment of the one or more items (20) by means of the at least one element (62) of the at least one impeller (60).

12. The method according to claim 11, wherein the method further comprises the steps of:
- keeping the at least one second electrode (40) stationary; and/or
- moving the at least one impeller (60) with and/or around the at least one first electrode (30) during at least part of the plasma treatment of the one or more items (20).

13. The method according to claim 11 or 12, wherein the method further comprises the step of:
- moving the at least one element (62) of the impeller (60) by means of a rotational movement with and/or around the at least one first electrode (30).

14. The method according to one or more of the claims 11 to 13, wherein the method further comprises the steps of:
- providing the element (62) of the at least one impeller (60) comprising and/or consisting of:
- the at least one first electrode (30); and/or
- the at least one insulating dielectric barrier (50).

15. A method according to one or more of the claims 8 to 14, wherein the method is a method for cleaning and/or sterilizing and/or disinfecting and/or inactivating pathogens and/or decontaminating the one or more items (20) in the reactor container (10) during the plasma treatment.
